# EUROPEAN PATENT APPLICATION

(11) **EP 1 310 795 A1**
(43) Date of publication of application: **14.05.2003**
(21) Application number: 01957012.6
(22) Date of filing: 21.08.2001
(51) Int. Cl.: G01N 33/566

(54) **CHEMICAL SENSOR AND METHOD OF DETECTING CHEMICAL**

(30) Priority: 25.08.2000 JP 2000256319
(71) Applicant: Sanyo Electric Co., Ltd., Moriguchi-shi, Osaka-fu 570-8677 (JP)
(72) Inventor: IKEMATSU, Mineo, Moriguchi-shi, Osaka 570-8677 (JP)
(74) Representative: Denison, Christopher Marcus
(86) International application number: JP0107168
(87) International publication number: WO02016945

(57) **Abstract**

A chemical substance sensor is formed by arranging a sensor chip on a microcartridge formed with a micropassage and further arranging a prism on this sensor chip. In the sensor chip, a metal thin film is formed on one surface of a substrate, and an immobilizer is further formed thereon. In order to detect a chemical substance with the chemical substance sensor, a solution containing a sample and odorant binding protein is fed to the micropassage for immobilizing the odorant binding protein to the immobilizer. The chemical substance contained in the sample is detected on the basis of the quantity of immobilization of the odorant binding protein.

## Description

### Technical Field

The present invention relates to a chemical substance sensor detecting a chemical substance contained in a sample and a method of detecting a chemical substance.

### Background Art

Detection of an odorant is performed as one of control tests for the quality of an item such as food, for example. In this specification, a chemical substance causing an odor is generically referred to as an odorant. Detection of this odorant is employed for detecting an odor caused when prepared or reserved food or the like is deteriorated or detecting an odor of a container such as a PET bottle getting into food contained therein, for example. An attempt is made to detect the odor of powder forming a mine from the ground surface thereby locating the mine.

Such an odorant has been generally detected by employing a macromolecular substrate or a membrane, for example, and measuring change of the conductivity of the substrate or change of the membrane potential when the odorant adheres to the substrate or the membrane.

In such a method, however, it is disadvantageously difficult to distinguish bonding of the odorant from bonding of a substance other than the odorant. In order to avoid this problem, therefore, methods employing antibodies are developed ("J. E. Roederer and G. J. Bastiaans, Anal. Chem. 55 (1983) 2333.", "K. A. Davis and T. R. Leary, Anal. Chem. 61 (1989) 1227.", "H. Muramatsu et al., Anal. Chem., 59 (1987) 2760.", "M. Thompson et al., IEEE Trans. Ultrasonics, Ferroelectrics, Frequency Control, UFFC-34 (1987) 127.", "M. Thompson et al., Anal. Chem., 58 (1986) 1206." and "F. Caruso et al., Colloid Interface Sci. 178 (1996) 104."). That is, an antibody is immobilized to a macromolecular substrate or a membrane for bonding only a specific odorant to this antibody. The dielectric constant of the macromolecular substrate or the membrane potential changes when the specific odorant is bonded to this antibody, and hence the specific odorant is detected on the basis of this change so that bonding of non-specific substances can be eliminated.

A conventional odor sensor employing the aforementioned macromolecular substrate or the membrane generally has low sensitivity (up to ppm). Even if the sensitivity is high (up to ppb), substrate surface modification necessary for absorbing the odor is so deficient in variation (about 10-odd types at the most) that treatable odors have been limited.

In the detection method employing an antibody for the odorant, on the other hand, the antibody is so high-priced that it is costly to perform detection. In such a detection method employing the antibody, specificity in bonding between the antibody and the odorant is extremely high and hence it is necessary to prepare an antibody corresponding to each odorant in order to detect an odor formed by an extremely large number of substances in general. Therefore, a method of detecting a plurality of types of odorants with antibodies is impractical in consideration of the cost required for preparing the antibodies and thermal instability of the antibodies.

### Disclosure of the Invention

An object of the present invention is to provide a chemical substance sensor and a method of detecting a chemical substance capable of performing detection with high sensitivity at a low cost and applicable to a plurality of types of chemical substances having different bonding characteristics.

Various animals have olfactory organs for sensing and discriminating odors. The mechanism of such an olfactory organ is recently molecular-biologically analyzed. In this analysis, it has been clarified that an odorant is bonded with specific receptors present in epithelial cells of an olfactory mucosa or the like, and a signal is transmitted into the epithelial cells through the receptors for detecting and discriminating the odorant.

In detailed clarification of the mechanism of the series of detection and recognition of the odorant, protein bonded with the odorant (hereinafter referred to as odorant binding protein) is discriminated and isolated ("J. Pevsner et al., Science, 241 (1988) 336."). The odorant binding protein can be bonded not only to the odorant but also to a specific chemical substance having no odor.

As to biological action, it is presumed that this odorant binding protein has a function of being bonded with an odorant, carrying this odorant to receptors on epithelial cells and transferring the odorant to the receptors. This odorant binding protein has also been biochemically analyzed and proved to have wide-ranging bonding force, peaked on bonding force with respect to a certain odorant, for substances similar to this odorant ("J. Pevsner et al., J. Biolog. Chem. 265 (1990) 6118.", "M. A. Bianchet et al., Nature Struc. Biolog. 3 (1996) 934." and "M. Tegoni et al., Nature Struc. Biolog. 3 (1996) 863"). In particular, such odorant binding protein also includes that having a cloned gene (the aforementioned J. Pevsner et al.), and can also be subjected to mass production.

The inventor has noted such odorant binding protein, and deeply studied on a method of detecting a chemical substance with this odorant binding protein. As a result, the inventor has found it possible to detect an odorant having a constant width in high sensitivity by utilizing odorant binding protein. The inventor has devised the present invention as follows:

A chemical substance sensor according to an aspect of the present invention comprises a cartridge having a passage capable of feeding a solution containing a sample and odorant binding protein, an immobilizer capable of coming into contact with the solution in the passage and arranged along a flow of the solution for immobilizing the odorant binding protein contained in the solution, and a detector that detects the quantity of immobilization of the odorant binding protein contained in the solution to the immobilizer on the basis of physical change of an interface between the solution containing the sample and the odorant binding protein and the immobilizer.

In order to determine presence/absence of a chemical substance in a sample with the chemical substance sensor according to the present invention, the immobilizer is first arranged on the cartridge as described above. A solution containing only odorant binding protein without containing a chemical substance is fed into the passage of the cartridge for immobilizing the odorant binding protein contained in the solution to the immobilizer. The quantity of immobilization of the odorant binding protein immobilized in the aforementioned manner is detected by the detector on the basis of physical change of the interface between the solution and the immobilizer. The obtained quantity of immobilization of the odorant binding protein is employed as a reference value for comparison described later.

After detecting the quantity of immobilization of the odorant binding protein as to the solution containing no chemical substance in the aforementioned manner, the odorant binding protein immobilized to the immobilizer is removed.

Then, a solution containing a sample and odorant binding protein is fed into the passage of the cartridge, for immobilizing the odorant binding protein contained in the solution to the immobilizer. The quantity of immobilization of the odorant binding protein immobilized in the aforementioned manner is detected by the detector on the basis of physical change of the solution and the immobilizer. Further, the quantity of immobilization of the odorant binding protein obtained in this manner is compared with the aforementioned reference value.

In the aforementioned comparison of the quantity of immobilization of the odorant binding protein, the quantity of immobilization of the odorant binding protein immobilized to the immobilizer exhibits a value different from the reference value due to the chemical substance when the sample contains the chemical substance. When the value of the quantity of immobilization of the odorant binding protein thus changes as compared with the reference value, therefore, it is determined that the chemical substance has been detected. When the sample contains no chemical substance, on the other hand, the quantity of immobilization of the odorant binding protein immobilized to the immobilizer matches with the reference value. When the value of the odorant binding protein thus remains unchanged as compared with the reference value, therefore, it is determined that no chemical substance is detected.

When the aforementioned chemical substance is employed, as hereinabove described, presence/absence of the chemical substance in the sample can be readily determined on the basis of the quantity of immobilization of the odorant binding protein contained in the solution to the immobilizer, and the chemical substance contained in the sample can be detected with high sensitivity. Particularly in this case, a chemical substance having a molecular weight of not more than 200 can also be detected with high sensitivity. A method employing such a chemical substance sensor can be carried out at a lower cost as compared with the conventional method employing an antibody.

The odorant binding protein, peaked on bonding force with respect to a specific chemical substance, widely has bonding force also for chemical substances similar to this chemical substance. According to the aforementioned chemical substance sensor employing such odorant binding protein, therefore, chemical substances can be widely detected.

In the method employing such a chemical substance sensor, correlation is recognized between the affinity of the chemical substance and the odorant binding protein for each other and the quantity of immobilization of the odorant binding protein to the immobilizer. According to the method employing the aforementioned chemical substance sensor, therefore, the affinity between the chemical substance and the odorant binding protein can be obtained on the basis of the quantity of immobilization of the odorant binding protein, and the odorant can be discriminated and specified on the basis of difference of this affinity.

In the method employing such a chemical substance sensor, correlation is recognized between the quantity (concentration) of the chemical substance bonded to the odorant binding protein and the quantity of immobilization of the odorant binding protein to the immobilizer. According to the method employing the aforementioned chemical substance sensor, therefore, the quantity (concentration) of the chemical substance contained in the sample can be measured, i.e., the chemical substance present in the sample can be determined on the basis of the quantity of immobilization of the odorant binding protein.

In the above, the physical change may be change of a refractive index.

When the odorant binding protein contained in the solution is adsorbed to the immobilizer and immobilized, the refractive index on the interface between the solution and the immobilizer changes. Therefore, immobilization of the odorant binding protein can be sensed and the quantity of immobilization can be detected by detecting such change of the interface between the solution and the immobilizer.

The detector may include a condenser arranged on the immobilizer, an irradiator that irradiates the interface with light through the condenser, a refractive index measuring device that measures the refractive index by reflected light from the interface through the condenser, an immobilization quantity calculator that calculates the quantity of immobilization of the odorant binding protein to the immobilizer on the basis of the change of the refractive index measured on the basis of the refractive index measuring device, and a determiner that determines presence/absence of the chemical substance in the sample on the basis of the quantity of immobilization calculated by the immobilization quantity calculator.

The quantity of immobilization of the odorant binding protein can be obtained on the basis of change of the refractive index on the interface between the solution and the immobilizer by employing the aforementioned detector. Therefore, presence/absence of the chemical substance in the sample can be determined on the basis of this quantity of immobilization.

The refractive index measuring device may include a light-transmitting substrate having the condenser arranged on one surface, a metal thin film arranged between the other surface of the substrate and the immobilizer, a photoreceptor that receives the reflected light from the interface, and a resonant angle measuring device that measures a resonant angle in surface plasmon resonance on the basis of an output from the photoreceptor thereby measuring change of the refractive index by the reflected light from the interface.

The irradiator may irradiate the interface with monochromatic light through the condenser. In particular, the irradiator may irradiate the interface with a parallel component of the monochromatic light through the condenser.

The condenser may be a prism. The cartridge may be a microcartridge having a micropassage.

The odorant binding protein may have a dimer structure. In particular, the odorant binding protein may be bovine derivation odorant binding protein. Such bovine derivation odorant binding protein widely has affinity for terpenoid, esters, aldehydes, aromatic series etc. Therefore, the aforementioned wide-ranging chemical substances can be sensed with high sensitivity by employing such bovine derivation odorant binding protein for the aforementioned chemical substance sensor.

A method of detecting a chemical substance according to another aspect of the present invention comprises steps of arranging an immobilizer for immobilizing odorant binding protein contained in a solution on a cartridge having a passage capable of feeding the solution containing a sample and the odorant binding protein so as to come into contact with the solution in the passage along a flow of the solution, feeding the solution containing the sample and the odorant binding protein into the passage, detecting the quantity of immobilization of the odorant binding protein to the immobilizer on the basis of physical change of an interface between the solution containing the sample and the odorant binding protein and the immobilizer, and analyzing a chemical substance contained in the sample on the basis of the detected quantity of immobilization of the odorant binding protein.

In the method of detecting a chemical substance according to the present invention, the solution containing the sample and the odorant binding protein is fed into the passage of the cartridge for immobilizing the odorant binding protein contained in the solution to the immobilizer. When the odorant binding protein is thus immobilized to the immobilizer, physical change results on the interface between the solution and the immobilizer. In the method of detecting a chemical substance according to the present invention, therefore, the quantity of immobilization of the odorant binding protein is detected on the basis of such physical change.

The quantity of such immobilization of the odorant binding protein to the immobilizer varies with presence/absence of a chemical substance in the sample. In this case, therefore, presence/absence of the chemical substance in the sample can be detected on the basis of change of the quantity of immobilization of the odorant binding protein.

In the aforementioned method of detecting a chemical substance, as hereinabove described, presence/absence of the chemical substance in the sample can be determined on the basis of change of the quantity of immobilization of the odorant binding protein to the immobilizer, and the chemical substance in the sample can be detected with high sensitivity. Particularly in this case, a chemical substance having a molecular weight of not more than 200 can also be detected with high sensitivity. Such a method of detecting a chemical substance can be carried out at a lower cost as compared with the conventional method employing an antibody.

The odorant binding protein, peaked on bonding force with respect to a specific chemical substance, widely has bonding force also for chemical substances similar to this chemical substance. According to the aforementioned method of detecting a chemical substance employing such odorant binding protein, therefore, chemical substances can be widely detected.

The step of analyzing may include a step of comparing the quantity of immobilization of the odorant binding protein in a sample containing no chemical substance with the detected quantity of immobilization of the odorant binding protein and determining presence/absence of the chemical substance on the basis of the result of the comparison.

In this case, the quantity of immobilization of the odorant binding protein in the sample containing no chemical substance is employed as a reference value for determining presence/absence of the chemical substance in the sample by comparison with this reference value. In other words, it is determined that the chemical substance has been detected when the value of the detected quantity of immobilization of the odorant binding protein is different from the aforementioned reference value, while it is determined that no chemical substance is detected when the detected quantity of immobilization of the odorant binding protein matches with the aforementioned reference value.

In the aforementioned method, the physical change may be change of a refractive index.

When the odorant binding protein contained in the solution is adsorbed to the immobilizer and immobilized, the refractive index on the interface between the solution and the immobilizer changes. Therefore, immobilization of the odorant binding protein can be sensed and the quantity of immobilization can be detected by detecting such change of the interface between the solution and the immobilizer.

The step of analyzing may include a step of analyzing affinity between the chemical substance contained in the sample and the odorant binding protein on the basis of the detected quantity of immobilization of the odorant binding protein and discriminating the chemical substance contained in the sample on the basis of this affinity.

In the aforementioned method of detecting a chemical substance, correlation is recognized between the affinity of the chemical substance and the odorant binding protein for each other and the quantity of immobilization of the odorant binding protein to the immobilizer. According to the aforementioned method, therefore, the affinity between the chemical substance and the odorant binding protein can be obtained on the basis of the quantity of immobilization of the odorant binding protein, and the chemical substance can be discriminated and specified on the basis of the difference of this affinity.

The step of analyzing may include a step of determining the chemical substance contained in the sample on the basis of the detected quantity of immobilization of the odorant binding protein.

In the aforementioned method of detecting a chemical substance, correlation is recognized between the quantity (concentration) of the chemical substance bonded to the odorant binding protein and the quantity of immobilization of the odorant binding protein to the immobilizer. According to the aforementioned method, therefore, the quantity (concentration) of the chemical substance in the sample can be measured, i.e., the chemical substance present in the sample can be determined on the basis of the quantity of immobilization of the odorant binding protein.

The odorant binding protein may have a dimer structure. In particular, the odorant binding protein may be bovine derivation odorant binding protein. The bovine derivation odorant binding protein widely has affinity for terpenoid, esters, aldehydes, aromatic series etc. Therefore, the aforementioned wide-ranging chemical substances can be sensed with high sensitivity by employing the bovine derivation odorant binding protein for the aforementioned method.

The detecting step may include steps of irradiating the interface with light through a condenser, measuring a refractive index by reflected light from the interface through the condenser, calculating the quantity of immobilization of the odorant binding protein to the immobilizer on the basis of change of the measured refractive index, and determining presence/absence of the chemical substance in the sample on the basis of the calculated quantity of immobilization.

In this case, the quantity of immobilization of the odorant binding protein can be obtained on the basis of change of the refractive index on the interface between the solution and the immobilizer. Therefore, presence/absence of the chemical substance in the sample can be determined on the basis of this quantity of immobilization.

### Brief Description of the Drawings

Fig. 1 is a schematic diagram showing a method of detecting an odorant according to an embodiment of the present invention.
Fig. 2 illustrates the principle of a chemical substance sensor shown in Fig. 1.
Fig. 3 is a schematic diagram showing a detailed structure of a sensor chip of the chemical substance sensor shown in Fig. 1.
Fig. 4 is a schematic diagram showing a state immobilizing odorant binding proteins (OBPs) to the sensor chip of the chemical substance sensor shown in Fig. 1.
Fig. 5 schematically illustrates the state of Fig. 4 immobilizing the OBPs.
Fig. 6 is a schematic diagram showing a state of immobilization of OBPs with reference to a sample containing an odorant having low affinity for the OBPs.
Fig. 7 schematically illustrates the state of Fig. 6 immobilizing the OBPs.
Fig. 8 is a schematic diagram showing a state of immobilization of OBPs with reference to a sample containing an odorant having high affinity for the OBPs.
Fig. 9 schematically illustrates the state of Fig. 8 immobilizing the OBPs.
Fig. 10 illustrates the relation between the affinity of OBPs and odorants for each other and diffusion coefficients of the OBPs in solutions.
Fig. 11 illustrates a process of forming an OBP gene in Inventive Example.
Fig. 12 illustrates results of measurement of the quantities of immobilization of OBP 6C in Inventive Example 1.
Fig. 13 illustrates results of measurement of the quantities of immobilization of OBP 6C in Inventive Example 2.

### Best Mode for Carrying Out the Invention

In the following description, a method of detecting a chemical substance according to the present invention is applied to detection of an odorant.

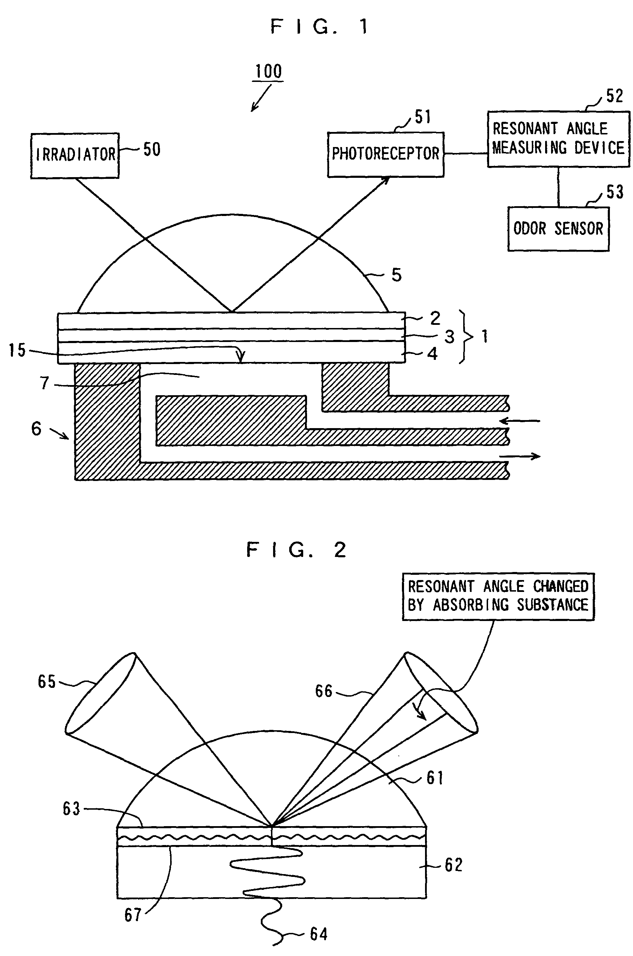

Fig. 1 is a schematic diagram showing a method of detecting an odorant according to an embodiment of the present invention.

In a chemical substance sensor 100 shown in Fig. 1, a sensor chip 1 is arranged on a microcartridge 6 formed with a micropassage 7, and a prism 5 is further arranged on this sensor chip 1.

The sensor chip 1 includes a substrate 2 made of a material such as glass, for example, transmitting light. The prism 5 is arranged on one surface of the substrate 2. The other surface of the substrate 2 is covered with a metal thin film 3 capable of forming surface plasmon resonance described later. This metal thin film 3 is made of a metal such as gold, silver or the like, so far as the metal is capable of forming surface plasmon resonance. In this case, the metal thin film 3 is made of gold (Au).

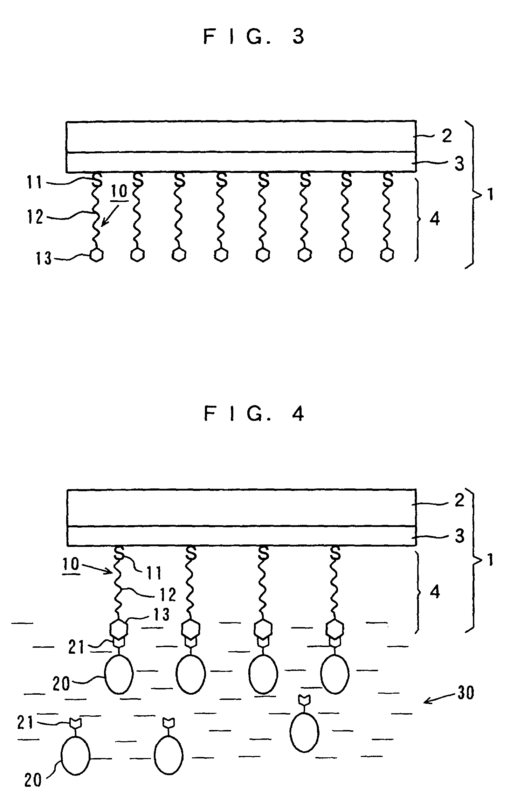

An immobilizer 4 is formed on the metal thin film 3. The details of the immobilizer 4 are described later with reference to Fig. 3.

The aforementioned chemical substance sensor 100 is provided with an irradiator 50 irradiating the substrate 2 with light through the prism 5. The irradiator 50 is formed to irradiate the substrate 2 with the light while changing the angle of incidence. The light emitted from this irradiator 50 is prepared from monochromatic light so that the refractive index can be simply measured. Further, the monochromatic light from this irradiator 50 is so formed that only a component parallel to an interface 15 is applied for forming surface plasmon resonance as described later.

A photoreceptor 51 receiving reflected light from the interface 15 is formed to align with the aforementioned irradiator 50. This photoreceptor 51 is provided with a resonant angle measuring device 52 and an odor sensor 53. The resonant angle measuring device 52 measures a resonant angle on the basis of the light reflected on the photoreceptor 51. The odor sensor 53 determines presence/absence of change of the refractive index of the interface 15 on the basis of the resonant angle measured in this manner for sensing change of the quantity of immobilization of odorant binding protein to the immobilizer 4.

This surface plasmon resonance (PM) method is described in detail in literature by P. B. Garland et. al (Quarterly Reviews of Biophysics 29, (1996) 91.). Fig. 2 shows the outline of this PM method.

When two transparent media 61 and 62 having different refractive indices come into contact with each other to form an interface 63 while a metal thin film layer is present therebetween and light is incident upon this interface 63 under a condition of totally reflecting the light from the side having a higher refractive index, light energy of part of the incident light penetrates into the side having a lower refractive index as an evanescent wave 64, as shown in Fig. 2.

An electric field component, referred to as surface plasmon (PM), perpendicular to the interface is formed in a portion of this metal thin film in the vicinity of the interface. When only a parallel component of monochromatic light is incident upon such an interface 63 as incident light 65 at a constant angle, PM 67 results from excitation by the aforementioned evanescent wave 64. The state resulting in such PM 67 is referred to as a resonance state, and incident light energy shifts to the PM 67 in this resonance state. Therefore, it follows that the intensity of reflected light 66 reflected by the interface 63 remarkably lowers due to the generation of the PM 67.

It has been clarified that the angle of incidence forming such a resonance state depends on a refractive index. Therefore, the angle of incidence forming such a resonance state is so measured that change of the refractive index can be measured from change of this angle. In this embodiment, change of the refractive index is measured through this principle.

The detailed structure of the sensor chip 1 is now described with reference to Fig. 3.

As hereinabove described, the immobilizer 4 is formed on the metal thin film 3 of the sensor chip 1. As shown in Fig. 3, the immobilizer 4 is formed by NTA linkers 10. These NTA linkers 10, having SH groups 11 on one ends, are fixed to the substrate 2 by bonding these SH groups 11 to Au contained in the metal thin film 3 of the substrate 2.

Further, the NTA linkers 10 is provided with hydrocarbon chains 12 on the centers. Steric exclusion between adjacent odorant binding proteins described later can be eliminated for bonding and fixing a large number of odorant binding proteins due to such provision of the hydrocarbon chains 12. The odorant binding proteins described later are softly fixed due to employment of the hydrocarbon chains 12, whereby the association ratio with the odorant binding proteins can also be improved. Therefore, the hydrocarbon chains 12 employed here can be preferably used regardless of the length thereof and presence/absence of side chains so far as the same attain such an effect.

Further, those having hydrophilic substituents and high affinity for water can be further preferably used as such hydrocarbon chains 12. In general, linkers repel a solution as hydrophobicity thereof is increased, leading to a possibility of rounding themselves. In order to release the linkers with respect to the odorant binding proteins, therefore, high flexibility is required to the linkers. Such high flexibility of the linkers can be implemented by improving hydrophilicity of the linkers.

As linkers having the aforementioned properties, commercially available kits (by Quagen) or sensor chips (Sensor Chip NTA by Biacore) can be simply used.

The aforementioned NTA linkers 10 are provided with NTAs (nitrilotriacetic acids) 13 on the other ends. These NTAs 13 contain chelated nickel (Ni). Nickel of these NTAs 13 and histidine in histidine tags of the odorant binding proteins are bonded with each other due to interaction. Thus, the odorant binding proteins are immobilized to the NAT linkers 10.

While the odorant binding proteins are immobilized through the bonding force between histidine and the NTAs 13. the odorant binding proteins can alternatively be immobilized to the linkers through combination of biotin and avidin (or streptoavidin) or combination of GST (glutathione S transferase) and glutathione or the like in place of the combination of histidine and the NTAs 13.

In order to detect an odorant with the chemical substance sensor 100 as shown in Fig. 1, an aqueous solution containing ethanol (the aqueous solution containing ethanol is hereinafter referred to as an ethanol solution) containing odorant binding proteins is first introduced into the micropassage 7 of the microcartridge 6. This ethanol solution is introduced along arrow in Fig. 1 in this case.

In this case, bovine derivation odorant binding protein (OBP; odorant binding protein, J. Pevsner et. al., J. Biolog. Chem. 265 (1990) 6118) having a dimer structure is employed as the odorant binding protein. This bovine derivation odorant binding protein is hereinafter referred to as OBP. A histidine tag constituted by six histidines is set on the OBP employed here, as described later.

The ethanol solution containing the OBPs is introduced into the micropassage 7 as described above, for feeding this solution into the micropassage 7 at an extremely small flow velocity of 1 µl/min. Thus, the OBPs contained in the ethanol solution 30 are immobilized onto the immobilizer 4 of the sensor chip 1. The state of immobilization of the OBPs is now described with reference to Fig. 4.

As described above, OBPs 20 contained in the ethanol solution 30 have histidine tags 21 each constituted by six histidines. These histidine tags 21 are formed by being fused with the OBPs 20. When the ethanol solution 30 containing the OBPs 20 is fed into the micropassage 7 at an extremely low velocity as described above, the histidine tags 21 of the OBPs 20 and nickel of the NTAs 13 interact so that the OBPs 20 are consequently immobilized (adsorbed) to the NTA linkers 10 of the immobilizer 4. Thus, partial OBPs 20 contained in the ethanol solution 30 are immobilized to the immobilizer 4.

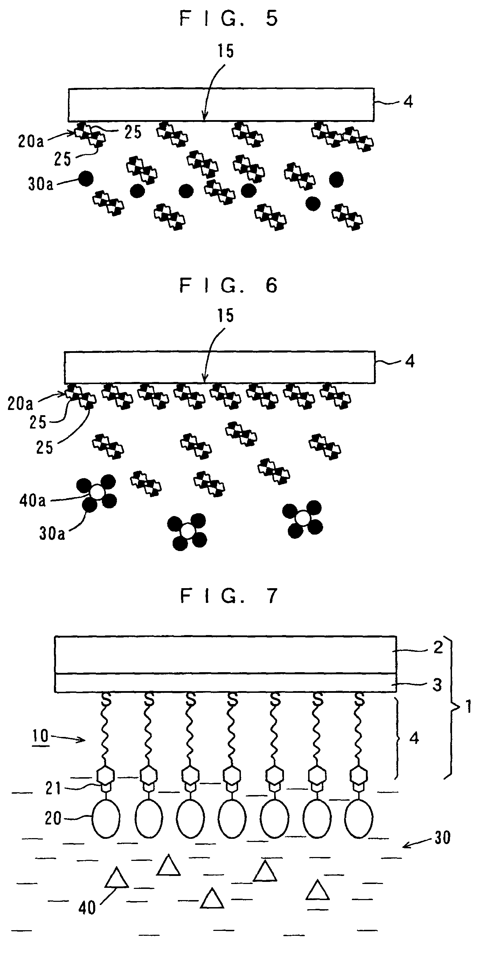

Fig. 5 schematically illustrates the state of the OBPs immobilized to the immobilizer as described above in states of molecules. As shown in Fig. 5, OBP molecules 20a have such a dimer structure that protein molecules 25 of monomers are bonded by noncovalent bonding. Parts of the OBP molecules 20a present here are immobilized to the immobilizer 4. On the other hand, the remaining OBP molecules 20a are not immobilized to the immobilizer 4 but present in the solution along with ethanol molecules 30a.

In the detection method according to this embodiment, the quantity of such immobilization of the OBPs 20 to the immobilizer 4 in the ethanol solution 30 containing only the OBPs 20 is first measured.

The refractive index of the interface 15 in the state shown in Figs. 4 and 5 is obtained from the resonance angle, for obtaining the quantity of immobilization of the OBPs 20 on the basis of this refractive index. In this case, the resonance angle measuring device 52 measures the resonance angle on the basis of the reflected light received in the photoreceptor 51, while the odor sensor 53 converts the measured value to a refractive index. The odor sensor 53 records the value of the refractive index obtained in this manner as the reference value for the quantity of immobilization of the OBPs 20. After the quantity of immobilization of the OBPs 20 is measured in the aforementioned manner, the OBPs 20 immobilized to the immobilizer 4 are washed away and removed.

Then, in the chemical substance sensor 100 shown in Fig. 1, an ethanol solution containing OBPs and a sample injected therein is introduced into the micropassage 7 while emitting light from the irradiator 50 toward the interface 15. The ethanol solution subjected to injection of the sample is fed into the micropassage 7 at an extremely small flow velocity of 1 µl/min. similarly to the above case. The sample injected into the ethanol solution may be air, or a solid dissolved into the ethanol solution.

As to the aforementioned ethanol solution containing the sample, the quantity of immobilization of the OBPs 20 to the immobilizer 4 is measured. In other words, the ethanol solution containing the sample and the OBPs 20 is fed into the micropassage 7, for obtaining the refractive index of the interface 15 in this case from the resonance angle and obtaining the quantity of immobilization of the OBPs 20. The odor sensor 53 records the quantity of immobilization of the OBPs obtained in this manner. Further, the quantity of immobilization of the OBPs obtained here is compared with the aforementioned reference value, for determining that odorants have been detected when difference is observed between the same and the reference value while detecting that no odorants are detected when no difference is observed between the same and the reference value.

When odorants are present in the sample injected into the ethanol solution, the quantity of immobilization of the OBPs 20 immobilized to the immobilizer 4 is increased or reduced as compared with the aforementioned case (Figs. 4 and 5) containing only the OBPs, due to the presence of the odorants. The state of the interface 15 changes following such change of the quantity of immobilization of the OBPs 20, and hence the resonance angle changes in this case as compared with the aforementioned case (Figs. 4 and 5) containing only the OBPs. The odorants contained in the sample can be detected by detecting such change of the resonance angle as the change of the refractive index.

When no odorants are present in the sample, on the other hand, the quantity of immobilization of the OBPs 20 immobilized to the immobilizer 4 is similar to the quantity of immobilization in the aforementioned case (Figs. 4 and 5) containing only the OBPs, and hence the state of the interface 15 is similar to the aforementioned case (Figs. 4 and 5) containing only the OBPs in this case. Therefore, no change of the resonance angle is observed but the value of the refractive index converted from the resonance angle matches with the aforementioned reference value in this case. When the refractive index matches with the reference value in this manner, it is determined that no odorants are detected.

The mechanism of varying the quantity of immobilization of the OBPs 20 to the immobilizer 4 with the presence of the odorants is now described.

Fig. 6 is a diagram for illustrating a case where the sample contains odorants having low affinity for the OBPs. Fig. 6 shows each substance in the state of a molecule.

As shown in Fig. 6, odorants 40a contained in the sample have low affinity for the OBPs 20a, and hence the odorants 40a are not bonded with the OBPs 20a but the ethanol molecules 30a substitutionally surround the peripheries of the odorants 40a and keep the odorants 40a solubilized in this case.

The ethanol molecules 30a have a function of increasing the viscosity of the solution and inhibiting motion of the OBPs 20a. Diffusion (movement) of the OBPs 20a in the solution is suppressed and immobilization of the OBPs 20a to the immobilizer 4 is suppressed due to such function of the ethanol molecules 30a.

When the sample contains the odorants 40a having low affinity for the OBPs 20a as described above, the ethanol molecules 30a disturbing diffusion (movement) of the OBPs 20a are used for solubilization of the odorants 40a as hereinabove described, and hence the viscosity of the solution in the micropassage 7 (Fig. 1) is reduced. In this case, therefore, mobility of the OBPs 20a in the solution is increased and the diffusion coefficient of the OBPs 20a in the solution is increased.

When the sample contains odorants having low affinity for the OBPs 20a, the OBPs 20a readily move in the solution as hereinabove described, whereby the quantity of immobilization of the OBPs 20a immobilized to the immobilizer 4 is increased as compared with the case (Fig. 5) of only the OBPs with no odorants. Therefore, the odorants contained in the sample can be detected by detecting such increase of the quantity of immobilization of the OBPs 20a as the change of the resonance angle and further detecting the same as the change of the refractive index.

Fig. 7 is a schematic diagram for illustrating the state of Fig. 6 immobilizing the OBPs to the immobilizer in detail. As shown in Fig. 7, the OBPs 20 having the histidine tags 21 are bonded to the linkers 10 of the immobilizer 4 at a high ratio in this case. On the other hand, odorants 40 are not bonded to the OBPs 20 but present in the ethanol solution 30.

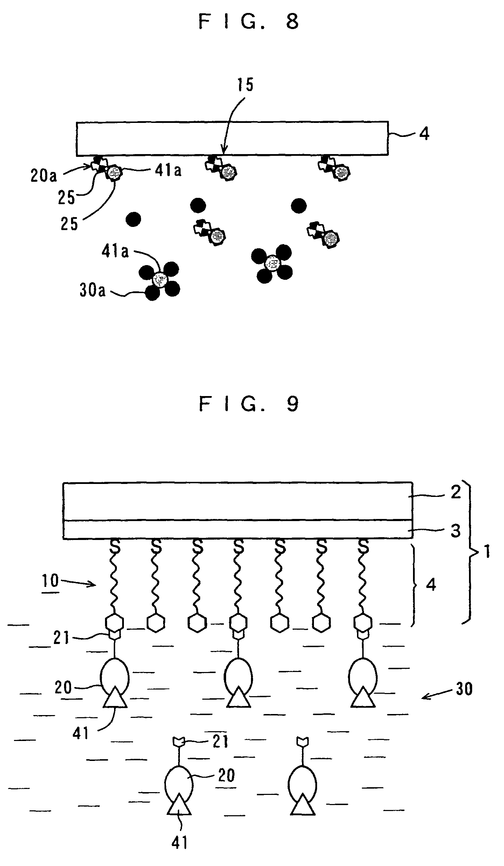

On the other hand, Fig. 8 is a diagram for illustrating a case where the sample contains odorants having high affinity for the OBPs. As shown in Fig. 8, odorants 41a contained in the sample have high affinity for the OBPs 20a, and hence the odorants 41a are readily bonded to the OBPs 20a in this case.

The odorants 41a and the OBPs 20a are bonded to each other as described above, whereby the ethanol molecules 30a having been used for solubilization of the odorants, i.e., the ethanol molecules 30a having surrounded the peripheries of the odorants 41a are detached from the odorants 41a. The viscosity of the solution in the micropassage (Fig. 1) is increased due to increase of such free ethanol molecules 30a. Therefore, the mobility of the OBPs 20a is reduced in this case. The OBPs 20a bonded with the odorants 41a become spheroids, and hence the mobility is further reduced in such OBPs 20a.

As described above, the mobility of the OBPs 20a in the solution is reduced and the diffusion coefficient of the OBPs 20a in the solution is reduced when the sample contains odorants having high affinity for the OBPs 20a. Thus, the quantity of immobilization of the OBPs 20a immobilized to the immobilizer 4 is reduced as compared with the case (Fig. 5) of only OBPs with no odorants in this case. Therefore, the odorants in the sample can be detected by detecting such reduction of the quantity of immobilization of the OBPs 20a as the change of the resonance angle and further detecting the same as the change of the refractive index.

Fig. 9 is a schematic diagram for illustrating the state of Fig. 8 immobilizing the OBPs to the immobilizer. As shown in Fig. 9, odorants 41 are bonded to the OBPs 20 having the histidine tags 21 in a high ratio in this case. In this case, however, the number of OBPs 20 bonded to the NTA linkers 10 of the immobilizer 4 is small.

As shown in the aforementioned Figs. 6 to 9, the odorants in the sample can be detected from the change of the quantity of OBP immobilization based on the change of the mobility (change of the diffusion coefficient) of the OBPs 20 and 20a whether the sample contains the odorants 40 and 40a having low affinity for the OBPs 20 and 20a or the sample contains the odorants 41 and 41a having high affinity for the OBPs 20 and 20a.

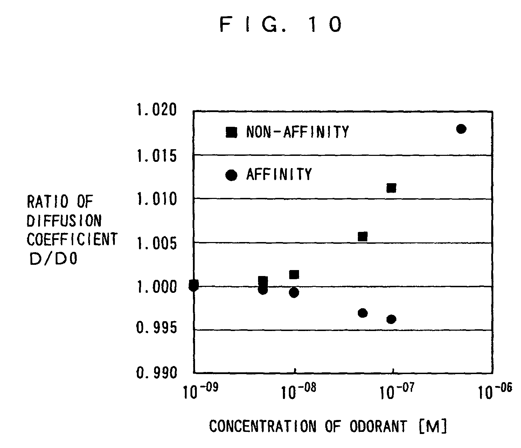

Fig. 10 is a diagram showing the relation between the affinity of OBPs and odorants for each other and diffusion coefficients of the OBPs in solutions. In this case, concentrations of the odorants are taken on the horizontal axis while the ratios (D/D₀) of the diffusion coefficients are taken on the vertical axis. D₀ on the vertical axis shows the diffusion coefficient of the OBPs in the case (Fig. 5) containing no odorants, and D on the vertical axis shows the diffusion coefficient of the OBPs in the case (Figs. 6 to 9) containing the odorants.

The case where the sample contains the (non-affinity) odorants having low affinity for the OBPs is first described. In this case, the quantity of ethanol used for solubilization of the odorants, i.e., for surrounding the odorants, is increased as the concentration of the odorants is increased, and hence the specific gravity of the solution is reduced and the viscosity of the solution is lowered. In this case, therefore, the mobility of the OBPs is enlarged and the diffusion coefficient D is increased as the concentration of the odorants is increased.

The case where the sample contains the (affinity) odorants having high affinity for the OBPs is now described. In this case, bonding between the OBPs and the odorants is prompted as the concentration of the odorants is increased within the range of not more than 10⁻⁷ M of the concentration of the odorants. Ethanol having surrounded the odorants is detached due to such bonding between the odorants and the OBPs, and hence the viscosity of the solution is so increased that the OBPs are hardly movable. The OBPs bonded with the odorants become spheroids, and hence the OBPs are further hardly movable. Thus, the diffusion coefficient D is reduced following increase of the concentration of the odorants within the range of not more than 10⁻⁷ M of the concentration of the odorants.

When the range of the concentration of the odorants exceeds 10⁻⁷ M, on the other hand, odorants not bonded with OBPs are present in access in addition to the odorants bonded with the OBPs. In this case, ethanol is used for solubilization of such excess odorants not bonded with the OBPs, i.e., for surrounding the odorants, and hence the viscosity of the solution is also lowered. In this case, therefore, the mobility of the OBPs bonded with the odorants is enlarged and the diffusion coefficient D is increased as the concentration of the odorants is increased.

In both of the non-affinity odorants and the affinity odorants, the diffusion coefficient D reaches the same value as the diffusion coefficient D₀ in the case containing no odorants in the range where the concentration of the odorants is not more than 10⁻⁹ M. This is because the concentration of the odorants is below the quantitative limit and hence the odorants exert no influence on movement of the OBPs.

The aforementioned method of detecting odorants based on the quantity of immobilization of the OBPs can implement high measuring sensitivity at least equivalent to that of the conventional method employing an antibody. Particularly in this case, high measuring sensitivity can be implemented also for odorants having molecular weights of not more than 200. Such a detection method, requiring no high-priced antibody, can be implemented at a low cost.

The OBP, peaked on bonding force with respect to a specific odorant, widely has bonding force also for odorants similar to this odorant. According to the aforementioned detection method employing the OBP, therefore, odorants can be widely detected.

In the OBP, bonding characteristics with respect to odorants can be readily changed by substitution of amino acid or the like. Thus , odorants can be discriminated and specified by changing the bonding characteristics of the OBP with respect to the odorants while detecting the odorants with a plurality of types of OBPs changed in the bonding characteristics respectively.

In the aforementioned method employing the OBPs, correlation is recognized between the affinity of the odorants and the OBPs for each other and the quantity of immobilization of the OBPs. According to the aforementioned method, therefore, the affinity between the odorants and the OBPs can be obtained on the basis of the quantity of immobilization of the OBPs, and the odorants can be discriminated and specified on the basis of difference in this affinity.

In the aforementioned method employing the OBPs, further, correlation is recognized between the quantity (concentration) of the odorants bonded to the OBPs and the quantity of immobilization of the OBPs. According to the aforementioned method, therefore, the quantity (concentration) of the odorants in the sample can be measured, i.e., the odorants present in the sample can be determined on the basis of the quantity of immobilization of the OBPs.

The bovine derivation protein (OBP) employed in the aforementioned method can be bonded with the following various terpenoids, cyclopentane and jasmine derivatives, esters, musks, aldehydes and aromatic series, and hence the same can be preferably utilized for sensing these wide-ranging odorants.

More specifically, the aforementioned terpenoids include dimethyl octanol, citralva, dihydromyrcenol and citronellol as those having high affinity for the aforementioned OBP and geranyl acetate, citronellal, citral dimethyl acetal, dimethyl octane, geranyl acetaldehyde, retinol, ionone, citronellyl acetate, dimethol, nerol, carbon, geraniol, linalool, menthone, retinal, dimethyl octhene, neo-alloocimen etc. as those having moderate affinity.

The aforementioned cyclopentane and jasmine derivatives include cisjasmone, jasmal, jessemal, bacdanol, methyl dihydrojasmonate, epimethyl dihydrojasmonate etc. as those having moderate affinity.

The aforementioned esters include benzyl isovalerate, benzyl benzoate etc. as those of high affinity, and include bornyl isovalerate, diethylphthalate, vertenex, octyl isovalerate, octyl isobutyrate, hexyl methylbutyrate etc. as those of moderate affinity.

The aforementioned musks include musk 89 as that of high affinity, and include coniferane, ambrettolide, galaxolide, cashmeran etc. as those of moderate affinity.

The aforementioned aldehydes include tetradecanal, amyl cinnamic aldehyde, undecanal and hexyl cinnamic aldehyde as those of high affinity, and include decanal, nonanal, heptanal, heptenal, citronellal, geranyl acetaldehyde, pinoacetaldehyde, pinylisobutyl aldehyde, cocal, myrmac aldehyde etc. as those of moderate affinity.

The aforementioned aromatic series include benzyl isovalerate, benzophenone, hexyl cinnamic aldehyde, hexyl pyridine, skatole etc. as those of high affinity, and include agrumea, eugenol, phenetyl alcohol etc. as those of moderate affinity.

Thus, the bovine derivation odorant binding protein (OBP) can be preferably utilized for specifically sensing these various odorants.

While the case of employing the bovine derivation odorant binding protein (OBP) has been described in the above, rat, rabbit, porcine, mouse, deer or feline derivation odorant binding protein (A. Felicioli et al. Life Chem. Reports, 11 (1994) 347.) can alternatively be employed in place of the bovine derivation protein.

The aforementioned odorant binding protein may not be naturally isolated protein but, if a gene coding the odorant binding protein is isolated or cloned, for example, protein obtained by translating this gene in *vitro* can also be employed.

It is also possible to employ the protein obtained by altering the base sequence of this gene coding the odorant binding protein and changing affinity for an odorant. As to the method of altering the gene, known point mutation introduction, random muter genesis PCR, a kunkel method or the like can be preferably utilized.

While the case of applying the method of detecting a chemical substance according to the present invention to detection of an odorant has been described in the above, the method according to the present invention is also applicable to detection of a chemical substance, other than an odorant, such as environmental hormone such as bisphenol A, for example.

While the case of dissolving the OBPs and the sample in the ethanol solution has been described in the above, a solvent other than ethanol can also be arbitrarily employed for the aforementioned detection method so far as the same is a solvent capable of dissolving OBPs and the solvent itself is an organic solvent readily dissolved in water. For example, methanol, DMF (N,N-dimethylformamide), DMSO (dimethylsulfoxide), acetonitrile or the like may be employed in the above in place of ethanol.

While the case of detecting the quantity of immobilization of the odorant binding proteins on the basis of the refractive index has been described in the above, the quantity of immobilization of the odorant binding proteins may be detected by a method other than the refractive index method so far as the method utilizes the micropassage.

### Inventive Examples

A method of preparing bovine derivation odorant binding protein (OBP) employed in Inventive Examples 1 and 2 described later is now described.

### 1. Synthesis of Odorant Binding Protein Gene

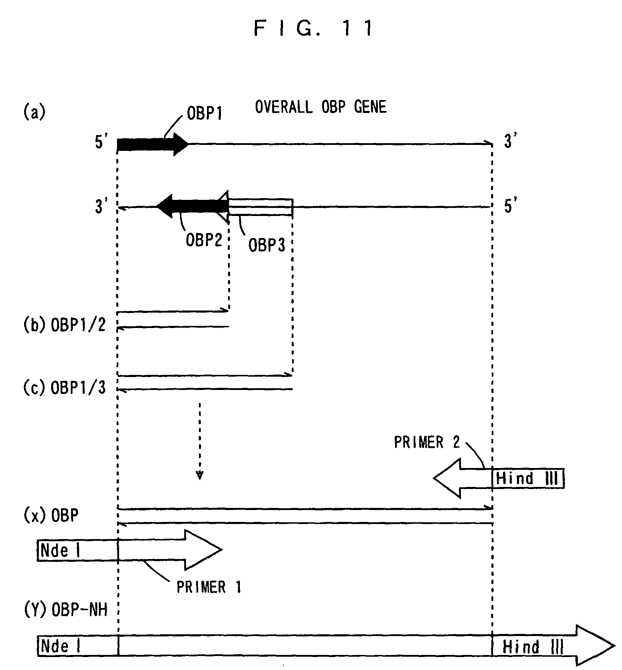

30 bases (this sequence is referred to as OBP 1, sequence number 1) from a five prime (5')-end most upstream the gene sequence of bovine derivation odorant binding protein (OBP) shown in Fig. 11 and 30 bases (OBP 2, sequence number 2) of downstream complementary sequence thereof including complementary sequence of 10 bases downstream the OBP 1 were employed for performing PCR under conditions shown in Table 1, for obtaining a fragment of 50 bases (OBP 1/2, sequence number 3).

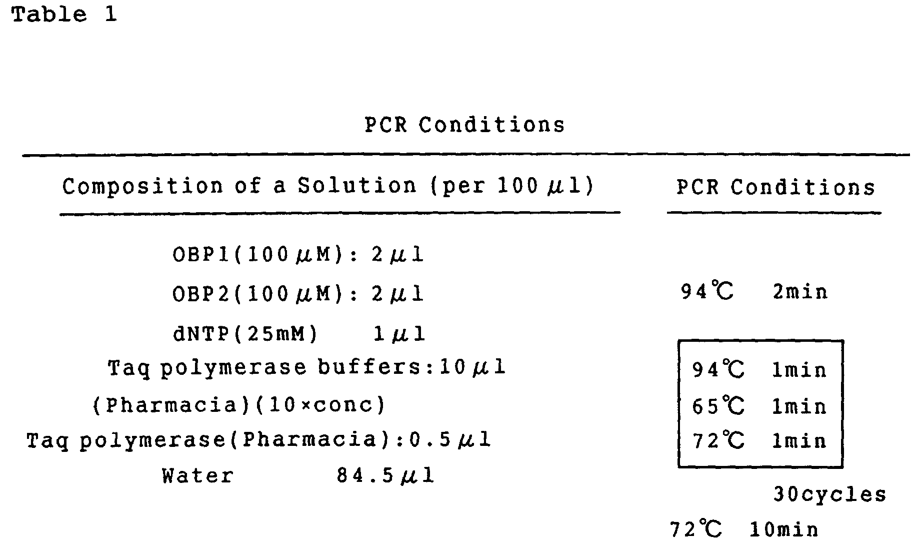

Similarly, 30 bases (OBP 3: sequence number 4) of complementary sequence including 10 bases downstream the OBP 2 were formed for performing PCR along with this OBP 1/2, for obtaining OBP 1/3.

The lengths of the synthetic fragments were successively elongated for finally synthesizing the whole length of the OBP gene.

### 2. Cloning of OBP to pET Vector and Transformation of Colon Bacillus BL21

An orthodromic primer 1 (sequence number 5) including the aforementioned restriction enzyme site Nde I sequence and an antidromic primer 2 (sequence number 6) including Hind III sequence were synthesized respectively in Fig. 11. The OBP gene was amplified by PCR with these primers 1 and 2, for consequently synthesizing OBP-NH having the Nde I sequence and the Hind III sequence on the respective ends (Fig. 11(Y)).

pET 28a (+) (by Novagen) was cut with Nde I and Hind III, and thereafter purified through a column (by Quiagen) to be dephosphorylated. The above OBP-NH and a pET indefinite vector were ligated for obtaining pETOBP-His (gene retention coding fusion protein having six histidines on a C-terminal of OBP).

Electroporation (by Biorad) was employed for introducing pETOBP-His into a competent cell of a colon bacillus BL21, for performing transformation. The transformed BL21/pETOBP was planted in an LB agarose plate containing kanamycin (30 µg/ml) and incubated at 37°C for a whole day and night, thereby obtaining a BL21 colony having pETOBP-His.

### 3. Mass Manifestation and Purification of OBP

The BL21/pETOBP colony was transferred to a 5 ml LB medium (kan⁺) and shaken at 37°C for a whole day and night, for performing pre-culture. This pre-culture solution was transferred to an LB medium (kan⁺) of 200 ml, and subjected to shaking culture at 37°C for three hours. IPTG was added to reach 1 mM, and shaking culture was further performed for three hours.

This culture solution was centrifuged at 4°C and 3000 rpm for 30 minutes, and the supernatant was thereafter removed for keeping the remaining pellet frozen at -110°C for a whole day and night. This frozen pellet was left to stand on ice for 30 minutes, and thereafter re-suspended in 10 ml of a bacteriolytic buffer. Lysozyme was added to this suspension to reach 1 mg/ml, and the suspension was left to stand on ice further for 30 minutes. Thereafter the suspension was dipped in ice water and on/off operations were repeated in an ultrasonic crusher every 10 seconds for crushing the cell for two minutes. The treated cell sap was centrifuged at 4°C and 17000 rpm for 30 minutes and slowly rotated at 4°C for one hour while adding Ni-NTA agarose (by Qiagen) to the supernatant. This supernatant was charged in a column and thereafter washed with a washing buffer (4 ml) twice, and the OBP was eluted in an elution buffer (500 µl) six times.

Table 2 shows the compositions of buffers employed in Inventive Example 3.

**Table 2**

| Composition of buffers | |
|---|---|
| Bacteriolytic buffer | 50mM NaH2PO4,pH8.0 |
| | 300mM NaCl |
| | 10mM imidazole |
| Washing buffer | 50mM NaH2PO4,pH8.0 |
| | 300mM NaCl |
| | 20mM imidazole |
| Elution buffer | 50mM NaH2PO4,pH8.0 |
| | 300mM NaCl |
| | 250mM imidazole |

OBP (hereinafter referred to as OBP 6C) having six histidines on the C-terminal was prepared in the aforementioned manner. The following Inventive Examples 1 and 2 were carried out with this OBP 6C.

### [Inventive Example 1]

In the present Inventive Example, a discrimination experiment was made with odorants of cineol, linalyl acetate, pinene, geraniol, citronellal and oxidized citronellal having substantially equal molecular weights. The details are now described.

In the present Inventive Example, an OBP 6C solution was prepared with the OBP 6C prepared in the aforementioned manner along with preparation of odorant solutions.

In preparation of the OBP 6C solution employed in the present Inventive Example, the OBP 6C prepared in the aforementioned manner was dissolved in an EB (eluent buffer) and diluted so that the concentration of the solution reached 200 nM. Table 3 shows the composition of the EB employed here.

**Table 3**

| Composition of the EB(eluent buffer) | | |
|---|---|---|
| 10mM | HEPES | |
| 150mM | NaCl | |
| 50µM | EDTA | pH 7.4 |
| 0.005% | Tween20 | |

In preparation of the odorant solutions employed in the present Inventive Example, on the other hand, the six types of odorants, i.e., cineol, linalyl acetate, pinene, geraniol, citronellal and oxidized citronellal were dissolved in ethanol solutions (EtOH) respectively, for preparing odorant solutions of 10⁻⁵ M in concentration as to the respective odorants.

100 µl of the OBP solution having the concentration of 200 nM prepared in the aforementioned manner and 0.1 µl of each odorant solution having the concentration of 10⁻⁵ M were mixed with each other, for preparing six types of sample solutions employed for measurement. In this case, the final concentration of the odorant in each sample solution is 10⁻ ⁸ µM.

An ethanol solution of the OBP 6C (final concentration of EtOH: 0.1 %) containing no odorant was also prepared as a comparative sample (blank).

Each sample solution prepared in the aforementioned manner was left to stand at 25°C for one hour.

In the present Inventive Example, the respective sample solutions were not simultaneously prepared but the individual sample solutions were prepared when executing immobilization of the OBP 6C described later.

Then, an NTA sensor chip (by Biacore) was set in BiacoreX (by Biacore) and thereafter the temperature was set to 25°C for performing priming with an elution buffer shown in Table 4 described later. Thereafter stabilization of the base was confirmed and the elution buffer was continuously fed at a flow velocity of 20 µl/min. until the temperature reached the set value.

Further, a reproduced solution (30 µl) shown in Table 4 described later was added at a flow velocity of 10 µl/min. and excessive bivalent cation was removed. Then, an Ni solution (30 µl) shown in Table 4 described later was added at a flow velocity of 10 µl/min. for chelating Ni to NTA and immobilizing Ni.

Table 4 shows the compositions of the buffer and the respective compositions employed here.

**Table 4**

| Solutions employed for measurement of OBP bonding characteristics | |
|---|---|
| Elution buffer: | 10mM HEPES, pH7.4 |
| | 0.15M NaCl |
| | 50µM EDTA |
| | 0.005% Tween20 |
| Reproduced solution | 10mM HEPES, pH8.3 |
| | 0.15M NaCl |
| | 0.35M EDTA |
| | 0.005% Tween20 |
| | 10mM HEPES, pH7.4 |
| Ni solution | 0.15M NaCl |
| | 50µM EDTA |
| | 0.005% Tween20 |
| | 500µM NiCl₂ |

Then, one of the sample solutions (10 µl) prepared in the above was added at a flow velocity of 1 µl/min. for 10 minutes, for immobilizing the OBP 6C onto the sensor chip. After completion of immobilization, the quantity of immobilization of the OBP 6C was measured. Thereafter the reproduced solution shown in Table 4 was employed for washing and removing the OBP 6C immobilized onto the sensor chip.

Operations similar to the above were performed also as to the other sample solutions, for measuring the quantities of immobilization of the OBP 6C as to all of the six types of sample solutions and the comparative (blank) sample solution.

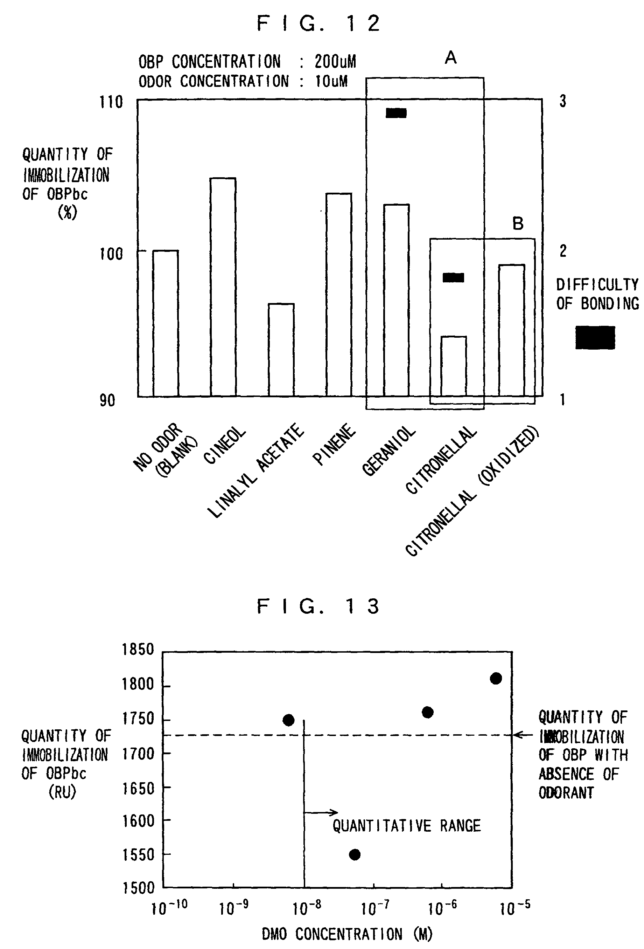

Fig. 12 shows the results of measurement of the quantities of immobilization of the OBP 6C in Inventive Example 1. In this case, the quantity of immobilization of the comparative sample (blank) containing no odorant was regarded as 100 % for calculating relative values of the quantities of immobilization of the OBP 6C in the respective samples with reference thereto.

As shown in Fig. 12, difference in affinity for the OBP 6C is reflected on the quantities of immobilization of the OBP 6C in the odorants, i.e., cineol, linalyl acetate, pinene, geraniol, citronellal and oxidized citronellal having substantially equal molecular weights. As to geraniol and citronellal, for example, citronellal has higher affinity for the OBP 6C as compared with geraniol, as shown in a range A in Fig. 12. Therefore, the quantity of immobilization of the OBP 6C is reduced in citronellal as compared with geraniol.

As shown in a range B in Fig. 12, affinity for the OBP 6C is reduced in oxidized citronellal as compared with non-oxidized citronellal. Therefore, the quantity of immobilization of the OBP 6C is increased in oxidized citronellal as compared with the non-oxidized case.

The affinity between the odorants and the OBP 6C is reflected on the quantities of immobilization of the OBP 6C as hereinabove described, and hence it has been clarified possible to discriminate the odorants on the basis of the difference in affinity.

### [Inventive Example 2]

In the present Inventive Example, the relation between the concentration of an odorant in a sample and the quantity of immobilization of the OBP 6C was studied. In this case, dimethyl octanol (DMO) was employed as the odorant.

In the present Inventive Example, an OBP 6C solution was first prepared along with preparation of DMO solutions. In this case, the OBP 6C solution was prepared by a method similar to the preparation method in Inventive Example 1. In preparation of the DMO solutions, DMO was dissolved in ethanol (EtOH), for preparing solutions of 10⁻³, 10⁻⁴, 10⁻⁵ and 10⁻⁶ M in concentration respectively.

100 µl of the OBP 6C solution having the concentration of 200 µM and 0.5 µl of each of the DMO solutions of 10⁻³, 10⁻⁴, 10⁻⁵ and 10⁻⁶ M, thereby preparing sample solutions having DMO final concentration values of 5 × 10⁻⁶, 5 × 10⁻⁷, 5 × 10⁻⁸ and 5 × 10⁻⁹ M respectively. Also in this case, an ethanol solution (final concentration of EtOH: 0.1 %) of the OBP 6C containing no DMO was prepared as a comparative sample, similarly to the case of Inventive Example 1.

The respective sample solutions prepared in the aforementioned manner were left to stand at 25°C for one hour.

In the present Inventive Example, the respective sample solutions were not simultaneously prepared but the individual sample solutions were prepared when executing immobilization of the OBP 6C as described later.

After preparing the sample solutions in the aforementioned manner, the quantities of immobilization of the OBP 6C were measured as to the respective sample solutions by a method similar to that in Inventive Example 1.

Fig. 13 illustrates the results of measurement of the quantities of immobilization of the OBP 6C in Inventive Example 2.

As shown in Fig. 13, constant correlation is recognized between the concentration of DMO and the quantity of immobilization of the OBP 6C in the range of the DMO concentration of at least 5 × 10⁻⁸ M. Thus, it has been clarified possible to determine the concentration of DMO from the quantity of immobilization of the OBP 6C when the concentration of DMO is at least 10⁻⁸ M.

When the concentration of DMO was smaller than 10⁻⁸ M, the concentration of DMO exceeded the quantitative limit and hence DMO could not be determined. It has been clarified that the quantity of immobilization of the OBP 6C is equalized with the quantity of immobilization (broken line in Fig. 13) with absence of the odorant in a concentration range exceeding such a quantitative limit.

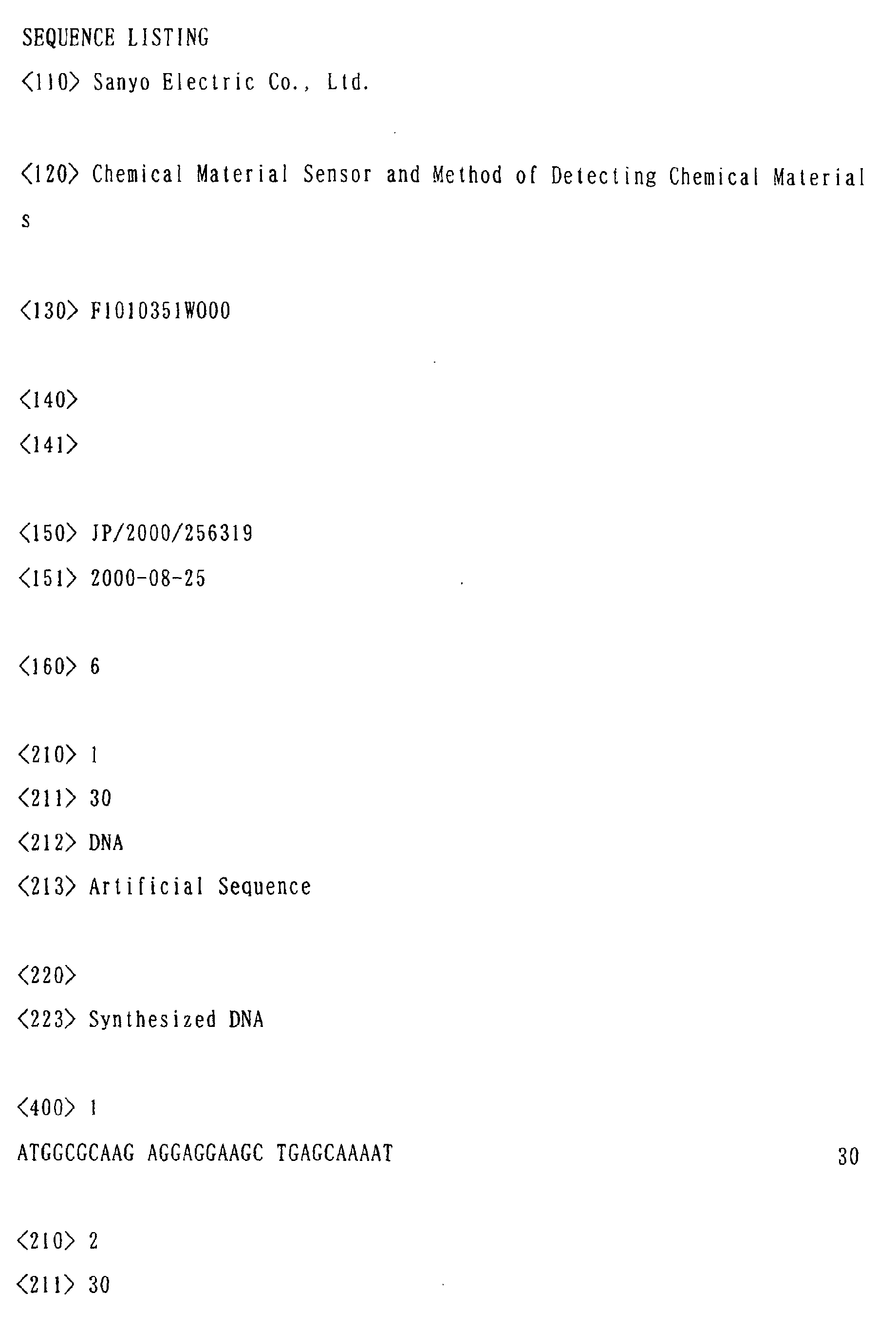

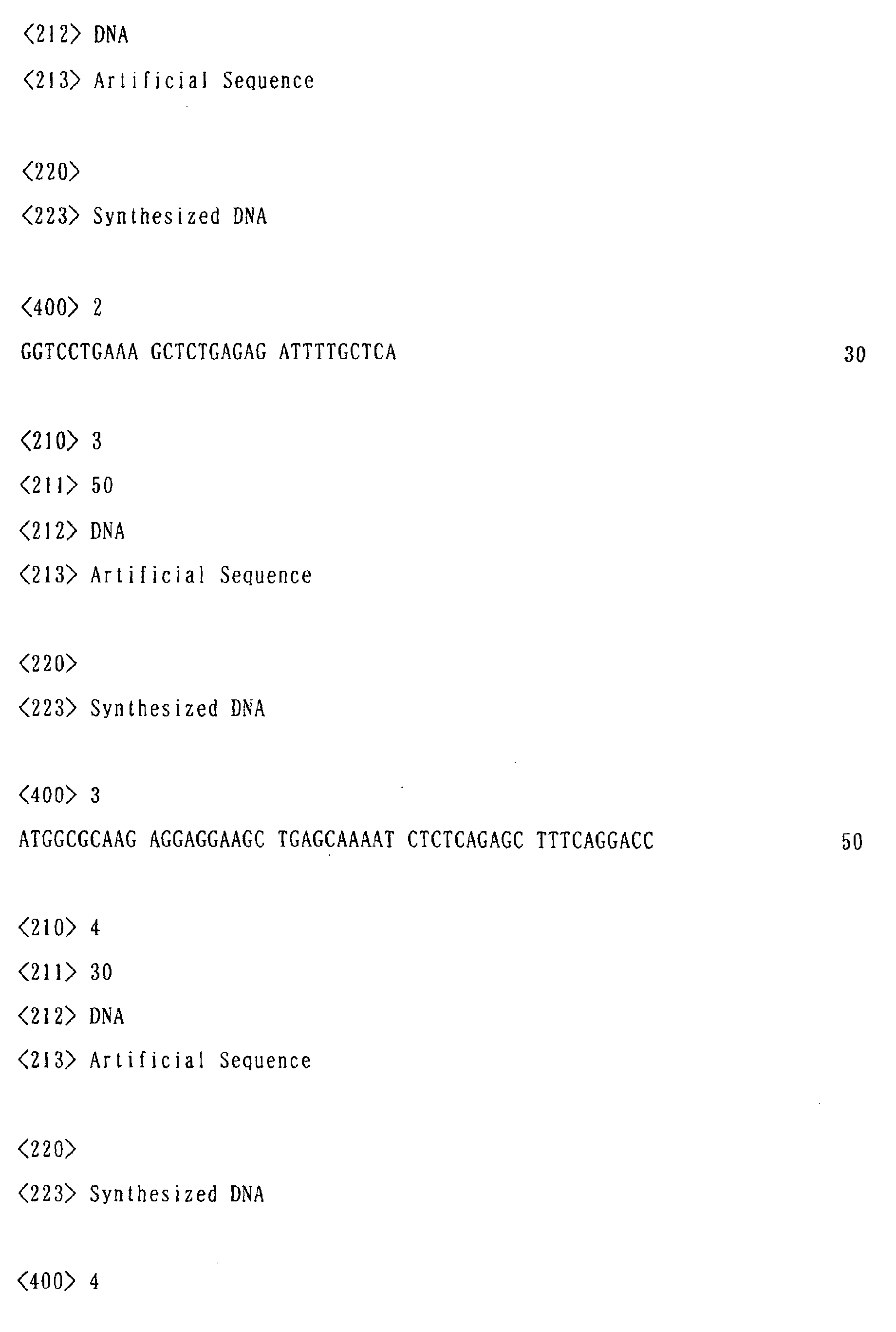

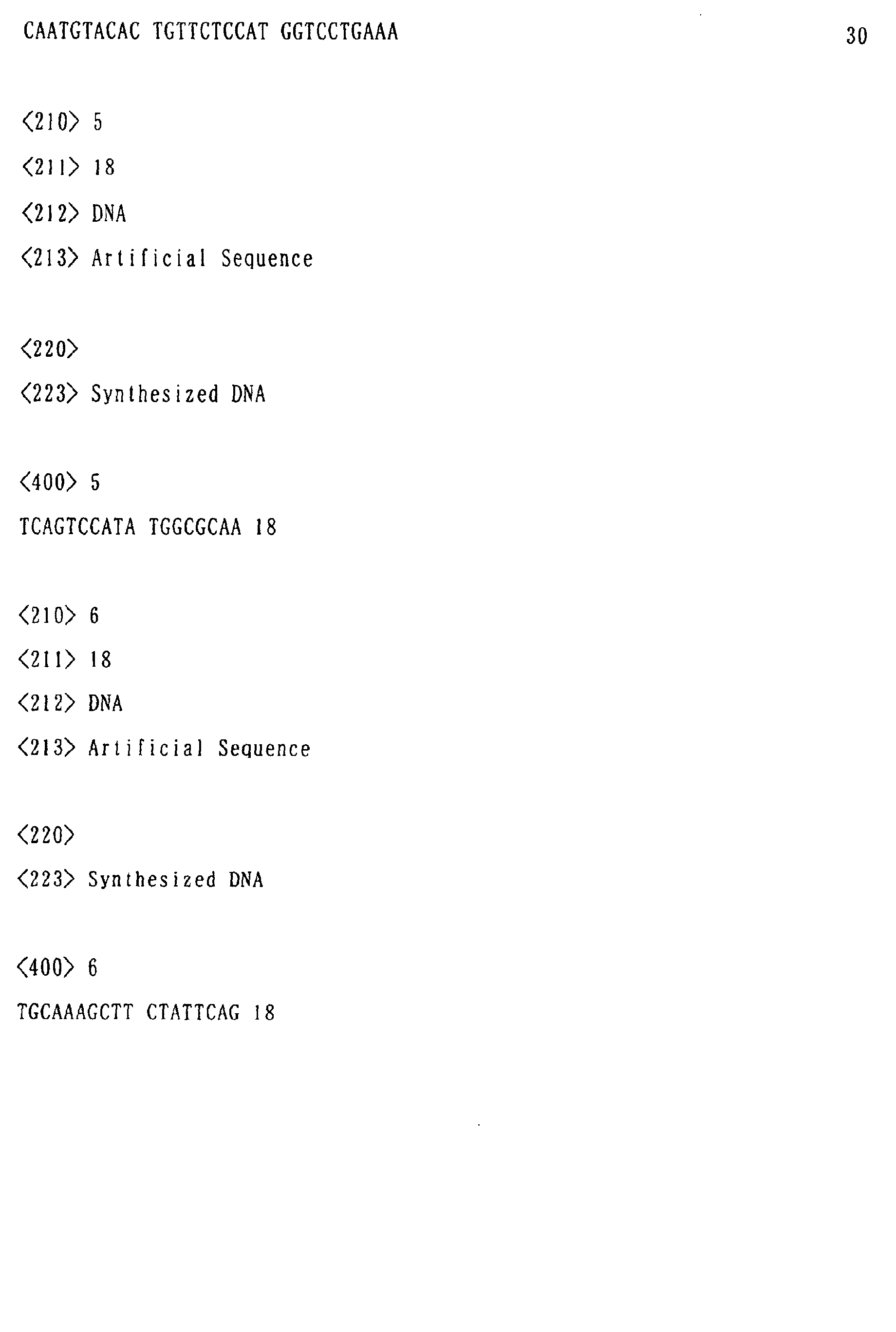

## Claims

1. A chemical substance sensor comprising:
a cartridge having a passage capable of feeding a solution containing a sample and odorant binding protein;
an immobilizer capable of coming into contact with said solution in said passage and arranged along a flow of said solution for immobilizing said odorant binding protein contained in said solution; and
a detector that detects the quantity of immobilization of said odorant binding protein contained in said solution to said immobilizer on the basis'of physical change of an interface between the solution containing said sample and said odorant binding protein and said immobilizer.

2. The chemical substance sensor according to claim 1, wherein said physical change is change of a refractive index.

3. The chemical substance sensor according to claim 2, wherein said detector includes:
a condenser arranged on said immobilizer,
an irradiator that irradiates said interface with light through said condenser,
a refractive index measuring device that measures the refractive index on the basis of reflected light from said interface through said condenser,
an immobilization quantity calculator that calculates the quantity of immobilization of said odorant binding protein to said immobilizer on the basis of the change of said refractive index measured by said refractive index measuring device, and
a determiner that determines presence/absence of a chemical substance in said sample on the basis of the quantity of immobilization calculated by said immobilization quantity calculator.

4. The chemical substance sensor according to claim 3, wherein said refractive index measuring device includes:
a light-transmitting substrate having said condenser arranged on one surface,
a metal thin film arranged between the other surface of said substrate and said immobilizer,
a photoreceptor that receives the reflected light from said interface, and
a resonant angle measuring device that measures a resonant angle in surface plasmon resonance on the basis of an output from said photoreceptor thereby measuring change of the refractive index by the reflected light from said interface.

5. The chemical substance sensor according to claim 3, wherein said irradiator irradiates said interface with monochromatic light through said condenser.

6. The chemical substance sensor according to claim 5, wherein said irradiator irradiates said interface with a parallel component of said monochromatic light through said condenser.

7. The chemical substance sensor according to claim 1, wherein said condenser is a prism.

8. The chemical substance sensor according to claim 1, wherein said cartridge is a microcartridge having a micropassage.

9. The chemical substance sensor according to claim 1, wherein said odorant binding protein has a dimer structure.

10. The chemical substance sensor according to claim 1, wherein said odorant binding protein is bovine derivation odorant binding protein.

11. A method of detecting a chemical substance comprising steps of:
arranging an immobilizer for immobilizing odorant binding protein contained in a solution on a cartridge having a passage capable of feeding said solution containing a sample and said odorant binding protein so as to come into contact with said solution in said passage along a flow of said solution;
feeding the solution containing the sample and said odorant binding protein into said passage;
detecting the quantity of immobilization of said odorant binding protein to said immobilizer on the basis of physical change of an interface between the solution containing said sample and said odorant binding protein and said immobilizer; and
analyzing a chemical substance contained in said sample on the basis of said detected quantity of immobilization of said odorant binding protein.

12. The method of detecting a chemical substance according to claim 11, wherein said analyzing step includes a step of comparing the quantity of immobilization of said odorant binding protein in a sample containing no chemical substance with said detected quantity of immobilization of the odorant binding protein to determine presence/absence of said chemical substance on the basis of the result of the comparison.

13. The method of detecting a chemical substance according to claim 11, wherein said physical change is change of a refractive index.

14. The method of detecting a chemical substance according to claim 11, wherein said step of analyzing includes a step of analyzing affinity between the chemical substance contained in said sample and said odorant binding protein on the basis of said detected quantity of immobilization of the odorant binding protein and discriminating the chemical substance contained in said sample on the basis of said affinity.

15. The method of detecting a chemical substance according to claim 11, wherein said step of analyzing includes a step of determining the chemical substance contained in said sample on the basis of said detected quantity of immobilization of the odorant binding protein.

16. The method of detecting a chemical substance according to claim 11, wherein said odorant binding protein has a dimer structure.

17. The method of detecting a chemical substance according to claim 11, wherein said odorant binding protein is bovine derivation odorant binding protein.

18. The method of detecting a chemical substance according to claim 11, wherein said step of detecting includes steps of:
irradiating said interface with light through a condenser,
measuring a refractive index by reflected light from said interface through said condenser,
calculating the quantity of immobilization of said odorant binding protein to said immobilizer on the basis of change of said measured said refractive index, and
determining presence/absence of the chemical substance in said sample on the basis of said calculated quantity of immobilization.
